# EUROPEAN PATENT SPECIFICATION

(11) **EP 1 591 783 B1**
(45) Date of publication and mention of the grant of the patent: **20.02.2008**
(21) Application number: 04010121.4
(22) Date of filing: 28.04.2004
(51) Int. Cl.: G01N 27/447, C07K 1/04, G01N 33/68, G01N 1/42

(54) **Method for detection and analysis of macromolecular complexes**
Verfahren zum Nachweis und zur Analyse von makromolekularen Komplexen
Méthode pour la détection et l'analyse des complexes macromoléculaires

(43) Date of publication of application: 02.11.2005
(73) Proprietor: MAX-PLANCK-GESELLSCHAFT ZUR FÖRDERUNG DER WISSENSCHAFTEN E.V., 14195 Berlin (DE)
(72) Inventor: Baumeister, Wolfgang, 81476 München (DE); Nickell, Stephan, 80469 München (DE); Kofler, Christine, 6600 Pinswang (AT); Boicu, Marius, 81377 München (DE); Keil, Thomas, 82319 Starnberg (DE)
(74) Representative: Vossius & Partner

(56) References cited:
- WO-A-02/075321
- WO-A-02/090966
- US-A- 5 858 781
- JETT STEPHEN D ET AL: "Snapshot blotting: Transfer of nucleic acids and nucleoprotein complexes from electrophoresis gels to grids for electron microscopy" PROCEEDINGS OF THE NATIONAL ACADEMY OF SCIENCES OF THE UNITED STATES OF AMERICA, vol. 91, no. 15, 1994, pages 6870-6874, XP002298562 ISSN: 0027-8424
- ROCKEL B ET AL: "Electron cryo-microscopy of VAT, the archaeal p97/CDC48 homologue from Thermoplasma acidophilum" JOURNAL OF MOLECULAR BIOLOGY, LONDON, GB, vol. 317, no. 5, 12 April 2002 (2002-04-12), pages 673-681, XP004472497 ISSN: 0022-2836
- SHOJI M ET AL: "Electrophoretic recovery of proteins from polyacrylamide gel" JOURNAL OF CHROMATOGRAPHY A, ELSEVIER SCIENCE, NL, vol. 698, no. 1, 28 April 1995 (1995-04-28), pages 145-162, XP004022956 ISSN: 0021-9673

## Description

The present invention relates to a method for the detection and/or analysis of the structure of macromolecular complexes and/or macromolecules comprising the steps of (a) purifying or separating said macromolecular complexes and/or macromolecules from a sample containing said macromolecular complexes and/or macromolecules by applying in a porous matrix a separation force in a first dimension (X-axis); (b) transferring in a second dimension (Z-axis) by adsorption forces the macromolecular complexes and/or macromolecules purified or separated in step (a) from the porous matrix onto a carrier wherein said carrier contacts the surface of the porous matrix and is positioned parallel to the surface of said matrix and parallel to the direction of the separation force applied in step (a); (c) immobilizing the macromolecular complexes and/or macromolecules on said carrier after transfer of step (b); and (d) assessing the structure of the macromolecular complexes and/or macromolecules on said carrier after immobilization of step (c).

Furthermore, the present invention relates to a method for analyzing whether a subject is afflicted by a disease or prone to developing a disease, wherein said disease is correlated with the presence or absence of protein complexes, and/or virus complexes, and/or bacterial complexes and/or fungal complexes, and/or prion-related complexes comprising the steps of (a) assessing for the presence and optionally the structure of said protein complexes, and/or virus complexes and/or bacterial complexes and/or fungal complexes and/or prion-related complexes by (aa) purifying or separating said protein complexes, and/or virus complexes, and/or bacterial complexes, and/or fungal complexes, and/or prion-related complexes from a sample putatively containing said protein complexes, and/or virus complexes and/or bacterial complexes and/or fungal complexes and/or prion-related complexes by applying in a porous matrix a separation force in a first dimension (X-axis); (ab) transferring in a second dimension (Z-axis) by adsorption forces the protein complexes, and/or virus complexes, and/or bacterial complexes, and/or fungal complexes, and/or prion-related complexes purified or separated in step (aa) from the porous matrix onto a carrier, wherein said carrier contacts the surface of the porous matrix and is positioned parallel to the surface of said matrix and parallel to the direction of the separation force applied in step (aa); (ac) immobilizing the protein complexes, and/or virus complexes, and/or bacterial complexes, and/or fungal complexes, and/or prion-related complexes on said carrier after transfer of step 2(ab); and (b) correlating the presence or absence or the structure of the protein complexes, and/or virus complexes, and/or bacterial complexes, and/or fungal complexes, and/or prion-related complexes on said carrier with the affliction of said subject by a disease or proneness to developing a disease.

The present invention also relates to a device for carrying out the method of the invention.

For manly biochemical needs it is desirable to be able to separate particular molecules from a mixture of molecules for further analysis. This includes for example the purification of proteins or other biomolecules from cell extracts; the purification of synthesized chemicals from contaminants or the separation of chemical mixtures. Further, separation of molecules is also important for structural analysis and identification of components of a mixture of molecules. In particular, the growing science of proteomics requires the identification of molecules and larger assemblies of interacting molecules forming macromolecular complexes within a cell's proteome that is, the entirety of proteins produced by a particular cell at a particular time. Therefore generic methods are needed that allow a quantitative analysis of large set of proteins having a variety of sizes, functions, activities, conformations and solubilities. The problem appears even more difficult with the requirements needed in pharmacological studies where hundreds to thousands of proteins and macromolecular complexes have to be analysed in parallel in a reasonable experimental time.

Traditionally such separation or purification has been accomplished by several different methods such as precipitation and dialysis techniques [Englard, S. et al. (1990); Pohl, T. (1990)], chromatography methods [Hagel, L. (1989); Stellwagen, E. (1990); Cutler, P. (1996)] and gel electrophoresis [Rickwood, D., et al. (1990); Hames, B.D. (1998)] which make use of the separation of molecules according to their mobility which is dominated by their charge/mass ratio. Electrophoresis is widely used and is usually performed in a porous matrix, such as agarose or polyacrylamide gel. Often molecules are separated by application of an electric field in a first dimension relative to their mobility. It is also possible to then carry out a second separation step by applying an electric field different in orientation from the electric field used for separation in the first dimension, thus creating a two dimensional gel electrophoresis. Thus, gel electrophoresis is a high resolution technique for separating mixtures of molecules.
Further analysis steps comprise a localisation and identification of the specific bands by staining methods such as Coomassie Blue, silver, ethidium bromide staining or with fluorescent compounds and the determination of the mass/charge respectively the mass by comparing the position of the bands with a reference marker running in parallel to the probe. Also the auto-fluorescent effect and the absorption of light can be used to localize and identify the specific bands. This procedure can be performed with native and denatured (SDS) gels.
Routinely the fractionated and purified molecules are investigated and characterized by mass-spectrometry or different blotting techniques such as Southern [Southern, E. (1975)] and Northern blots and specific probing with antibodies (Western blot). Prerequisite for further studies, in particular structural studies, is the extraction or separation of the already fractionated probe from the porous matrix, respectively the running gel. Cutting out the specific bands with a scalpel, blotting or shaking out the molecules of the gel are the standard techniques and commonly used. There are a variety of blotting methods known which include diffusion transfer, capillary transfer, heat-accelerated convectional transfer, vacuum blotting transfer and electroelution [Jeno, P. et al. (1996)]. The transfer method that is used most commonly for proteins is electroelution or electrophoretic transfer, because of its speed and transfer efficiency. This method uses the electrophoretic mobility of proteins to transfer them from the gel to a matrix. Electrophoretic transfer of proteins involves placing a protein-containing gel in direct contact with a piece of nitrocellulose or other suitable, protein-binding support and "sandwiching" this between two electrodes submerged in a conducting solution. After application of an electric field, the proteins move out of the polyacrylamide gel and onto the surface of the membrane, where the proteins become tightly attached. Most of the protein yield is therefore bound to the membrane and difficult to resolubilize in buffer solution. All these techniques use a solid carrier material like cellulose paper or a porous membrane where the separated probe is deposited.
A technique which conceptually resembles the electrophoretic filter blotting procedures and tries to apply electron microscopy is described in Jett & Bear, 1993, and derived from an earlier approach by Easom et al. 1989. In these publications an electron microscopical grid is used as a carrier suitable for the adsorption of the molecules on its surface and the examination in the electron microscope. This method is described as 'snapshot blotting' and includes several experimental steps such as the prestaining of the molecules with ethidium bromide or YOYO-1 (Molecular Probe), the special grid preparation procedure or the amount of glycerol (5-10%, vol/vol) which must be added prior to the loading of the samples onto the gel. Prior to mounting and inserting into the gel the grids are treated to increase their hydrophilicity and dipped into a spermidine buffer to render them positively charged. The electrophoretic gel is manipulated by removing it from the electrophoresis apparatus and placed on the transilluminator to visualize the bands. Vertical incisions are manually placed with a scalpel into the middle of each gel band to be sampled, then the gel is returned to the gel box and electrophoresed for 5 seconds. In a second step the gel is returned to the transilluminator, and the grids are inserted into the incisions at right angles to the top surface of the gel with the carbon side of the grid facing the negative poles. After the placement of the grids the gel is returned a second time to the gel box and electrophoresed for 5 seconds at 80 V and left in the gel for additionally 5 minutes with the power turned off. Then the grids are removed from the gel, fixed by negative staining in a standard manner and then further examined in a transmission electron microscope.
Experimentally all these steps are performed by hand, they are labor-intensive and prone to user error. There are significant changes of the electrophoretic properties of the separating media when the grids are inserted into the gels, leading to a loss of resolution. Also the process is susceptible to denaturing of the proteins by dehydration during the transport processes through air and by the fixation process at the grid's surface. Possible contaminations of the sample comes from the fact that rests of the separating gel sticks to the grid and undergoes chemical and physical changes in the electron microscope when exposed to the electron beam. Said changes can influence, for example, the melting, coagulation and charging properties of the samples to be analyzed. This results in a reduced quality of the electron micrographs and makes a quantitative and structural analysis of the proteins difficult.

An automated process is described in WO 02/090966 (Michael et al., 2002). It discloses an automated high-throughput system for excising spots or samples from an electrophoresis gel comprising a gel spot cutting device, use of holey carbon copper grids for electron cryo-microscopy, a computer controlled robotic arm, a sample plate handling system, with a computer connected to a scanning and imaging device to identify the spots to be excised by a cutting assembly and subsequently transferred to multiwell plates.

As discussed, the techniques and methods described above show major disadvantages with respect to the convenient and reliable detection and structural analysis of macromolecular complexes, in particular with respect to subsequent application to imaging technologies. The prior art did not even disclose methods that are easily handled and do not require labor intensive work. The technical problem underlying the present invention therefore was to provide such methods.

The solution to said technical problem is achieved by providing the embodiments characterized in the claims.

Accordingly, the present invention relates to a method for the detection and/or analysis of the structure of macromolecular complexes and/or macromolecules comprising the steps of (a) purifying or separating said macromolecular complexes and/or macromolecules from a sample containing said macromolecular complexes and/or macromolecules by applying in a porous matrix a separation force in a first dimension (X-axis); (b) transferring in a second dimension (Z-axis) by adsorption forces the macromolecular complexes and/or macromolecules purified or separated in step (a) from the porous matrix onto a carrier wherein said carrier is an electron microscopy grid and contacts the surface of the porous matrix and is positioned parallel to the surface of said matrix and parallel to the direction of the separation force applied in step (a); (c) immobilizing the macromolecular complexes and/or macromolecules on said carrier after transfer of step (b); and (d) assessing the structure of the macromolecular complexes and/or macromolecules on said carrier after immobilization of step (c).

The term "wherein said carrier contacts the surface of the porous matrix" as used in connection with the present invention means that macromolecular complexes can move or diffuse from the porous matrix to the carrier without any loss of structural information. This is usually achieved when a liquid layer forms in between the surface of the porous matrix and the surface of the carrier. Said liquid layer might for example immediately develop when the carrier is positioned on the surface of the porous matrix or might be made up by additionally applying the liquid.

The term "macromolecular complexes" means, in accordance with the present invention, any assembly made up of subunits. The subunit usually is the smallest unit of the macromolecular complex and is the cause for the characteristic structure or property of the macromolecular complex. The subunit is, for example, a protein monomer, a DNA- or RNA- macromolecule or an inorganic macromolecule.

The term "porous matrix" as used in the present invention, refers to an organic or inorganic medium characterized by its permeability for other substances.

The term "separation force" in connection with the present invention means the force a macromolecular complex or macromolecule is exposed to in an electric field resulting from its charged nature.

The term "adsorption force" as used in connection with the present invention means the force(s), responsible for a deposition of the macromolecular complexes on the the carrier. These forces include van der Waals forces, covalent forces, hydrogen bonds, dipole-dipole interactions, hydrophilic and hydrophobic effects.

The term "carrier" in accordance with the present invention implies any means that consists of a substrate which allows an adsorption of the macromolecular complexes and/or macromolecules and provides suitable imaging properties for e.g. electron microscopy.

As has been outlined above and in other terms the invention solves the recited technical problem by a highly reliable and reproducible method for detection and analysis of macromolecular complexes which can easily be handled. In particular as mentioned above, prior art methods rely on blotting techniques where macromolecular complexes, in particular proteins and protein complexes, are transferred from a porous matrix, such as a gel, on blotting membranes with the help of electroelution or electrophoretic transfer based on the electrophoretic mobility of the proteins. The macromolecular complexes, after being transferred on a blotting membrane, need to be resolved in e.g. buffer and subsequently transferred on a carrier suitable for direct imaging, as for example an electron microscopy grid. This procedure might lead in the end to the denaturing of the sample and/or result in a loss of material. Surprisingly, the inventors found that when placing the carrier parallel to the surface of the porous matrix, macromolecular complexes are transferred efficiently and without significant loss onto the carrier. After immobilization, the macromolecular complexes can be directly analysed. Furthermore, the native structure of the macromolecular complexes is preserved and the procedure is highly reliable and reproducible. The above finding is in particular surprising since no electrophoretic transfer steps or elution steps are needed for transferring the separated macromolecular complexes from the porous matrix onto the carrier. As mentioned, in the prior art the separated macromolecular complexes are cut out from the porous matrix, or blotted onto blotting membranes or shaken out of the porous matrix before they can be transferred on a carrier and fixed and subsequently analysed. The method of the present invention can efficiently be carried out without the above explained steps thus making the practical application easier, faster and also cheaper. As explained further above, it was known in the prior art that it is possible to place grids into vertical incisions in a right angle to the surface of the gel and subsequently transfer proteins onto said grids. This "snapshot blotting" technique requires a pre-treatment of the grids with spermidine buffer, furthermore, the gel has to be taken out from the electrophoresis apparatus in order to be able to visualize the bands where the incisions are then placed and then the gel is returned to the gel box and electrophoresed for 5 seconds at 80 V for transferring the proteins onto the electron microscopy grid. Placing the grid into the gel incisions and carrying out an electrophoretic step before fixing leads to many disadvantages in the subsequent analysis of the structure of the proteins in a transmission electron microscope, leading to a loss of resolution and possible contaminations from rests of the separation gel which sticks to the grid.
The most encouraging result obtained in accordance with the present invention was the demonstration that the above disadvantages could be overcome by the unexpected finding that a carrier, such as an electron microscopy grid, can be placed in contact parallel to the surface of a porous matrix and the transfer be effected without application of any additional electrophoretic transfer steps. It is particularly unexpected and surprising that the positioning of the carrier as effected in the method of the present invention, i.e. parallel to the surface of the porous matrix and parallel to the direction of the separation force, results in high quality representations of the structure of the macromolecular complexes.

Experimentally, a carrier, such as for example a commercially available electron microscopy grid, is placed on the spots of the porous matrix where the macromolecular complexes were identified beforehand by staining or autofluorescence. This identification is done by the user by making an image of the gel or can also be carried out with suitable computer software. The carrier is oriented parallel to the surface of the porous matrix therefore allowing a close contact between the carrier's and the matrix' surface. A drop of a suitable buffer solution, normally in the same pH range of the separated molecules to preserve native conditions, can optionally be applied onto the carrier and is dispensed between the carrier's and the matrix' surface. The macromolecular complexes and/or macromolecules move towards the carrier's surface which is covered by thin layer or film allowing the deposition of the molecules and which is transparent for the electron beam at the same time. A typical material for the above thin layer used in the field of electron microscopy and which is also applied in the present invention is a thin carbon layer, which can, optionally, have holes.
Usually, the carrier is removed from the surface of the porous matrix after 30 to 90 seconds. Optionally, to improve the yield of molecules deposited on the carrier's surface the gel can be roughened at the surface by pricking the gels surface with forceps or a razor-like device. In that way a larger amount of the macromolecular complexes inside the gel is exposed to the carriers surface and can be adsorbed. Another possibility to increase the amount of adsorbing molecules is to additionally apply an electric field between the carrier and the porous matrix. By doing this an additional electric force is applied leading to an increased transport and deposition of molecules on the carrier's surface. It is also possible to increase the amount of molecules which adsorb to the carrier's surface by applying to the surface chemical substances providing specific binding domains for the macromolecular complexes.

This can be achieved e.g. by incorporation of hydrophobic or hydrophilic, polar or non-polar groups. Thus a tailored immobilization of specific molecules and molecular arrangements can be obtained. Also the morphology of the carrier's surface can be adapted to improve the binding properties [Eckerskorn, Ch. et al. (1993)]. Instead of using a continuous foil a holey carbon foil can be applied to the carrier's surface. In this case the buffer solution with the solved molecules covers the grid and the holey areas in the holey carbon film at the same time. After a standard cryo-fixation procedure the buffer solution forms amorphous ice in the holes of the holey carbon foil thus immobilizing the molecule's structure. The native structure, in particular of macromolecular complexes and proteins, is preserved almost in its entirety in this way. This amorphous ice can also be penetrated by the electron beam and used for the imaging of the macromolecular complexes. All these steps can be performed easily by an automated system using standard components, as a controlling computer, a micro fluidic system a robotic device for carrier handling and a scanning device. After the preparation of the macromolecular complexes on the carrier the fixation or immobilization can take place in an automated device. For storage of ice-embedded samples standard liquid nitrogen dewars are routinely used.

The present invention also relates to a method for analyzing whether a subject is afflicted by a disease or prone to developing a disease, wherein said disease is correlated with the presence or absence of protein complexes, and/or virus complexes, and/or bacterial complexes and/or fungal complexes, and/or prion-related complexes comprising the steps of (a) assessing for the presence and optionally the structure of said protein complexes, and/or virus complexes and/or bacterial complexes and/or fungal complexes and/or prion-related complexes by (aa) purifying or separating said protein complexes, and/or virus complexes, and/or bacterial complexes, and/or fungal complexes, and/or prion-related complexes from a sample putatively containing said protein complexes, and/or virus complexes and/or bacterial complexes and/or fungal complexes and/or prion-related complexes by applying in a porous matrix a separation force in a first dimension (X-axis); (ab) transferring in a second dimension (Z-axis) by adsorption forces the protein complexes, and/or virus complexes, and/or bacterial complexes, and/or fungal complexes, and/or prion-related complexes purified or separated in step (aa) from the porous matrix onto a carrier, wherein said carrier is an electron microscopy grid and contacts the surface of the porous matrix and is positioned parallel to the surface of said matrix and parallel to the direction of the separation force applied in step (aa); (ac) immobilizing the protein complexes, and/or virus complexes, and/or bacterial complexes, and/or fungal complexes, and/or prion-related complexes on said carrier after transfer of step 2(ab); and (b) correlating the presence or absence or the structure of the protein complexes, and/or virus complexes, and/or bacterial complexes, and/or fungal complexes, and/or prion-related complexes on said carrier with the affliction of said subject by a disease or proneness to developing a disease.

In a preferred embodiment of the method of the present invention said subject is a human.

The above advantageous embodiments of the present invention are of particular interest. The method of the present invention can be applied to the analysis of diseases which are correlated with the presence or absence of protein complexes, and/or virus complexes, and/or bacterial complexes, and/or fungal complexes, and/or prion-related complexes. Thus, it is possible to detect and analyze a wide range of diseases such as diseases related to the malfunctioning of protein quality control (proteasomes or chaperones), neurodegenerative diseases characterized by the presence of protein aggregates. The protein, virus, bacterial fungal and/or prion related complexes can be isolated from any human body fluid, blood, urine, stool, saliva. In that way the present invention can also be used for drug screening in pharmacological studies.

In an additional preferred embodiment of the invention the method further comprises after step 1(a) or step 2(aa) and before step 1(b) or step 2(ab) step (a'), wherein a second separation force is applied in a third dimension (Y-axis) to the macromolecular complexes and/or macromolecules purified or separated in step 1 (a) or the protein complexes, and/or virus complexes, and/or bacterial complexes, and/or fungal complexes, and/or prion-related complexes purified or separated in step 2(aa).

In a more preferred embodiment of the invention the vector of the second separation force describes an angle in the third dimension to the vector of the separation force of step 1(a) or step 2(aa) in the range between 1 degree and 180 degree.

The term "vector" in connection with the present invention describes the orientation of the separation force applied.

It is preferred that the vector describes an angle of 90 degree.

By applying a second separation force, it is possible to further separate a mixture of proteins and/or macromolecular complexes depending on the size and structure. It is therefore possible to undertake "fine-tuned" separation of complex macromolecular mixtures.

In another preferred embodiment of the present invention said separation force is generated by an electric field or by gravity.

In yet another preferred embodiment of the present invention the porous matrix is stained and/or scanned after step 1 (a) or step 2(aa) and before step 1 (b) or step 2(ab).
In order to identify the macromolecular complexes of interest, it is possible to stain the porous matrix, in particular the gel, before transfer onto the carrier. The staining procedure is carried out according to standard protocols known in the art, such as ethidium bromide staining and silver staining methods [Wray, W. et al. (1981); Merril, C. et al. (1981); Williams, L. R. (2001)]

Alternatively, the porous matrix can also be scanned without a staining procedure, using the intrinsic fluorescence physical properties of the protein sample.

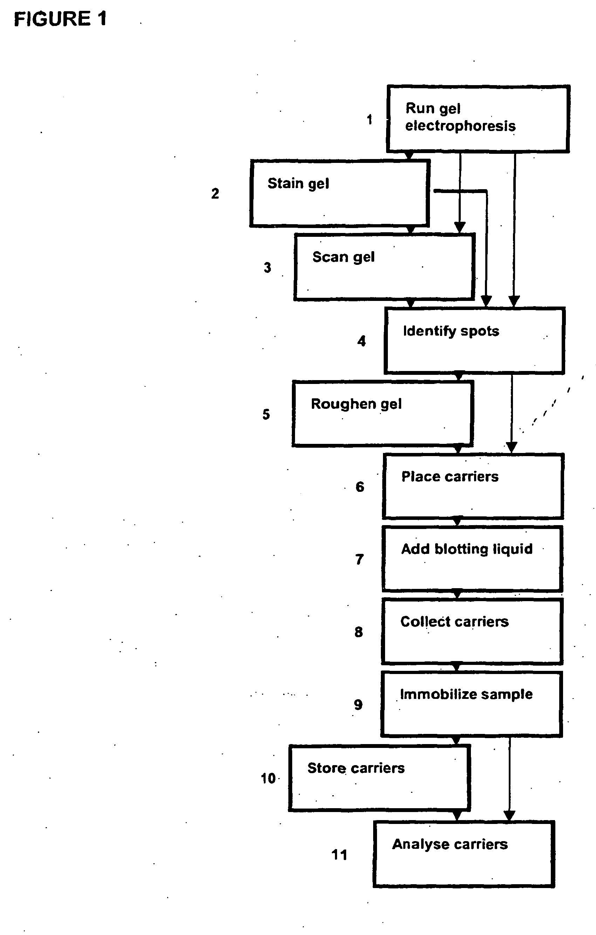

Usually, in the method of the present invention, as shown in Figure 1 and described in Examples 1.2 and 1.3, it is preferred that the staining procedure be carried out in the gel parts adjacent to the areas of interest of the gel which contain the bands with the macromolecular complexes.

In an additional preferred embodiment of the present invention the surface of the porous matrix is roughened.

This advantageous embodiment of the present invention, as also shown in Example 1.4, allows a more efficient transfer of the macromolecules of interest onto the carrier. In this respect, the porous matrix, in particular the gel, is pricked with forceps at the bands of interest to enlarge the surface of the gel and thus deeper gel layers with a high concentration of macromolecules become exposed to the surface of the porous matrix

In a further embodiment of the present invention the carrier which contacts the surface of the porous matrix is surrounded by a buffer medium.

As outlined in Example 1.6, it is possible to add a drop of running buffer medium onto the carrier after said carrier has been positioned on the porous matrix. This embodiment of the present invention is particularly useful in order to allow a diffusion driven movement of the macromolecules onto the carrier surface. This is particularly important when the humidity in the chamber surrounding the porous matrix or in the porous matrix itself is not allowing a liquid layer to form between the carrier and the surface of the porous matrix.

In an additional preferred embodiment of the present invention the transfer in step 1 (b) or step 2(ab) is carried out by an electric field wherein the vector of said electric field is oriented in the second dimension (Z-axis).

The Z-axis is preferably oriented perpendicular to the vector of the separation force (X-axis) and to the surface of the carrier or gel.

The transfer of the macromolecules of interest onto the carrier can be accelerated and the yield of transferred sample can be increased, by additionally applying an electric field. The gel is therefore applied to a conducting material, i.e. a metal plate and connected to the anode of an electric circuit. The carrier is connected to the cathode of the circuit. In this way an electric field is formed resulting in an electric force attracting the macromolecular complexes towards the carrier where they become adsorbed. In contrast to the concept of electroelution the carrier is used as an electrode and as a deposition surface for the sample at the same time.

In another preferred embodiment of the present invention, said porous matrix is a gel or a nanocomposite.
The term "nanocomposite" in connection with the present invention means a physical material which has component grains of 1 to 1000 nm in diameter, or has layers or filaments of the same range of thickness.

The porous matrices which can be used in connection with the present invention are of materials such as ceramic, metals (e.g. Aluminum), plastic and cellulose.

In a more preferred embodiment of the present invention the gel is a polyacrylamide gel or an agarose gel.

It is preferred in the method of the present invention that a polyacrylamide gel be used which is a standard in electrophoretic separation and provides separation capabilities for a wide range of samples.

In another more preferred embodiment of the present invention said gel is a gradient gel.

In a most preferred embodiment of the present invention said gradient is in the range of 0,01 % (w/v) to 50% (w/v) more preferably in the range of 0,5% (w/v) to 40%(w/v) and even more preferred in the range 1 % to 8% (w/v).

In a more preferred embodiment of the present invention said electron microscopy grid is covered by a electron transmitting layer or a holey foil.
The term "electron transmitting layer" in connection with the present invention means a thin film (typically 3-30 nm) working as a substrate surface for the sample and at the same time being transparent for the electron beam. To prevent charging effects the film is conductive.

The electron-transmitting layer used in the present invention preferably is a carbon foil. The holey foil preferably is a holey carbon foil.

In an additional preferred embodiment of the present invention said macromolecular complexes are selected from the group consisting of protein complexes, virus particles, eubacteriae, archaebacteriae, organic and anorganic chemical compounds.

In a preferred embodiment of the present invention, said protein complexes are native protein complexes.
Each individual step, i.e. in particular the separation, transfer and immobilization of the macromolecular complexes can be performed under non-denaturating conditions. The native structure of the complexes is thus preserved.

In another preferred embodiment of the present invention said immobilisation in step 1 (c) or step 2 (ac) is cryo-fixation. Cryo-fixation is particularly advantageous since the structure of the macromolecular structures is preserved better than the negative staining protocol [Dubochet, J. et al. (1982)].

In yet another preferred embodiment of the present invention said immobilisation in step 1 (c) or step 2 (ac) is negative staining with heavy atom salts.
Heavy atom salts that might be applied in connection with the present invention comprise uranyl acetate or uranylformate or phosphotungstate or ammoniummolybdate. It is however preferred that uranyl acetate be used in the present invention which is widely used in the field of electron microscopy [Harris, J. R. et al. (1991)].

In an additional preferred embodiment of the present invention, said assessment of the structure of the macromolecular complexes and/or macromolecules in step 1(d) or said assessment for the presence and optionally the structure of said protein complexes, and/or virus complexes and/or bacterial complexes and/or fungal complexes and/or prion-related complexes in step 2(a) is effected by optical means.

The term "optical means" in connection with the present invention comprises any means or method which can be used for imaging of the macromolecules. These comprise electron microscopy, light microscopy or scanning probe microscopy techniques such as atomic force microscopy or scanning near field optical microscopy.

In another aspect of the method of the present invention said optical means is electron microscopy or light microscopy.

For carrying out the method of the present invention, the electron microscope is particularly preferred for imaging the macromolecules and macromolecular complexes of interest. The resolution of the electron microscope in the angstrom to nanometer range is useful for analysing a wide range of macromolecular complexes in three dimensions, in particular also protein complexes, and/or virus complexes, and/or bacterial complexes, and/or fungal complexes, and/or prion-related complexes correlated with diseases.

The method of the invention might also be carried out by applying light microscopy techniques for imaging. Using a light transparent carrier, i.e. a glass slide, the sample can be examined by a standard light microscope resolving structures in the range of several 100 nm to several microns or using advanced super-resolution instruments resolving structures with several 10 nm. Additionally fluorescence labels can be applied to the sample and imaged in a standard fluorescent microscope.

In a preferred embodiment of the method of the present invention, said method is a high throughput method.

The present invention also relates to a device or robot for the detection and/or analysis of the structure of macromolecular complexes and/or macromolecules as defined in the present invention or for the analysis whether a subject is afflicted by a disease or prone to developing a disease as defined in the invention, wherein said device or robot comprises (a) a gel loading device, (b) a gel transporter, (c) a grid blotting robot with a grid clamping device, a positioning system, a gel scanner and optionally a gel roughening tool, (d) a fixation device, (e) a storage device; and (f) a computer controlling unit.

The documents cited in the present specification are herewith incorporated by reference.

### The figures show:

Fig. 1 shows a flow diagram of one possibility to carry out the experimental procedure for the present invention.
Fig. 2 shows an illustration of a possible setup of the electron microscopy carrier on the separating gel covered by a drop of buffer solution.

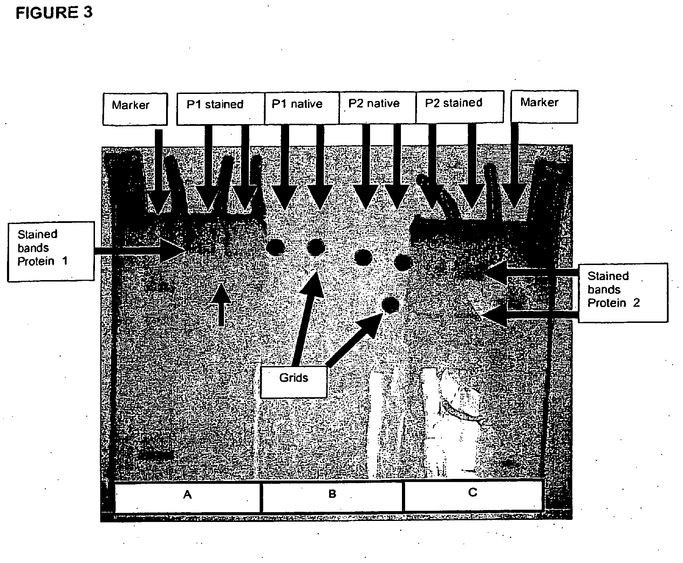

Fig. 3 shows a photograph of an electrophoretic, native gel which was loaded in multiple lanes with two different proteins P1 and P2. Protein P1 was loaded on the left half of the gel, P2 was loaded on the right half of the gel. The flanks are loaded with a marker ranging from a molecular weight between 20 kDa and 669 kDa. Area A and C are stained, area B is the non-stained region of the gel. Arrows top-down indicate the running direction of the gel in different lanes, the arrow left-right indicates the band of protein P1, the arrow right-left indicates the band of protein P2. The dark circles are the grids located in accordance with the present invention on the surface of the gel for blotting of the proteins P1 and P2 in the non-stained area B. The positioning of said grids is derived from the position of P1 or P2, respectively, located adjacent in the stained parts of the gel.
Fig. 4 shows electron micrographs of two examples of biological macromolecules (Proteasome and the AAA-ATPase VAT) separated and analyzed according to the present invention. Fig. 4A shows an electron micrograph of a negatively stained Proteasome, Fig. 4B shows the same protein under cryo-fixed conditions embedded in vitreous ice using a holey carbon film grid as a support. Fig. 4C shows an electron micrograph of a negatively stained VAT complex, Fig. 4D shows the same protein under cryo-fixed conditions embedded in vitreous ice using a holey carbon film grid.
Fig. 5 is an illustration of a possible automated setup of the process of the present invention which allows to handle pluralities of samples and grids.
Fig. 6 is an illustration of a possible device for the identification of the position of the separated macromolecular complex in the gel. The grid is then in accordance with the present invention placed on said identified position with the help of the xyz-positioning system controlled by a computer.

The examples illustrate the invention.

### Example 1

Example 1 illustrates one possible way for carrying out the invention. This is also shown in Figure 1.

### 1. Run gel electrophoresis at non-denaturing (native) conditions

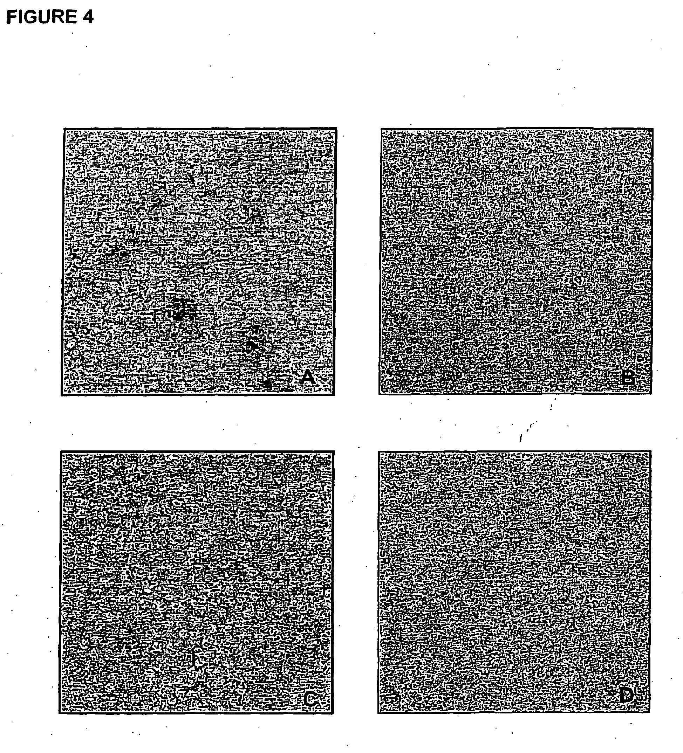

Electrophoresis of the macromolecular complexes to be analyzed, such as the ones shown in Figure 4, is performed under non-denaturing (native) conditions using a buffer system that maintains the native protein conformation, subunit interaction, and biological activity [Maurer, H. (1971); Laemmli, U. K. (1970)]. During native electrophoresis, proteins are separated based on their charge to mass ratios.
All gel electrophoresis steps are performed using equipment available at Invitrogen (Carlsbad, USA). A polyacrylamid separating gel (NuPAGE Novex® Tris-Acetate Gel, range 3-8%, 1 mm thick) is running in a (XCell SureLock) Mini-Cell at a constant Voltage of 150 V for 2.30 h with a current of 18 mA/gel (start) to 7 mA/gel (end). As a running buffer 100 ml 10X Tris-Glycine Native Running Buffer with 900 ml deionized water is used. In multiple lanes the same sample, here a macromolecular protein, is loaded to the gel to allow further identification (see step 3, below). To improve the identification step (see 3) one lane is loaded with a marker (HMW electrophoresis calibration kit 17-0445, Amersham Pharmacia Biotech, Piscataway, USA) which is well characterized by the manufacturer in terms of defined mass/charge ratios forming specific bands on the gel.

### 2. Stain gel

For visualization of the protein bands the gel is cut in half. One part is preserved in a wet tissue and stored for further usage in a fridge at 4°C. The other part is silver stained, based on the chemical reduction of silver ions to metallic silver on a protein band using SilverXpress® Silver Staining Kit available at Invitrogen (Carlsbad, USA).

### 3. Identify spots

An example for the identification of spots on the gel which contain the purified or separated macromolecular complexes is shown in Figure 3.

To identify bands of interest the stained part of the gel is positioned adjacent to the non-stained gel and carefully aligned at the top. By extrapolating the position of the stained bands to the non-stained gel the areas of interest, respectively the bands carrying macromolecules are located. For comparison and orientation additional information can be obtained from the marker lane showing well defined bands with well defined mass/charge ratios.

### 4. Roughen gel

After the identification of the position of the bands the gel can be pricked with a forceps at the bands of interest to enlarge the surface at these positions. In this way the top layer of the gel is disrupted and deeper gel layers with a high concentration of macromolecules are exposed to the surface of the gel. The depth of the disruption is half of the grids height, typically 0.5 mm, the lateral size is the diameter of the grid, typically 3 mm.

### 5. Place carriers

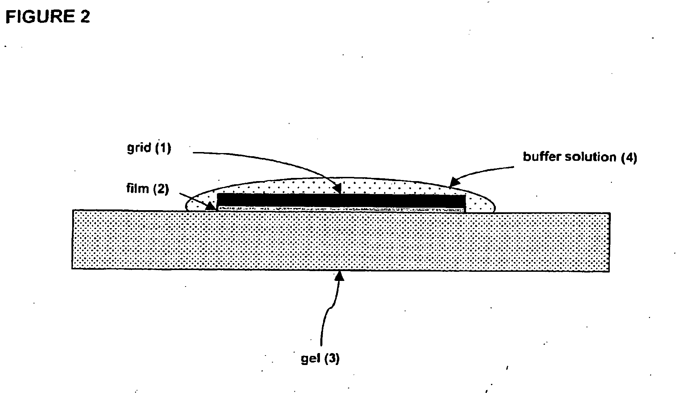

A copper grid covered with carbon film (commercially available at Plano GmbH, Wetzlar, Germany), respectively with a holey carbon film (commercially available at Quantifoil Micro Tools GmbH, Jena, Germany or Pacific GridTech, San Diego, USA) is placed centrally on specific, in step 3 identified bands with a forceps. The grid is handled carefully at the border to prevent any disruption of the carbon film and oriented in parallel to the gel's surface, and parallel to the direction to the direction of the separation force. Figure 2 shows schematically, how the grid is oriented on the gel.

### 6. Add blotting liquid

After the positioning of the grids an additional drop of buffer liquid is added to the grid to provide a diffusion medium between the gel and the grid surface. Therefore 10 µl running buffer solution is added to the grid with a pipette. The solution surrounds the gel and allows a movement of the macromolecules to the carbon layer on the grid surface. Here the proteins are adsorbed. An incubation time of around 60 seconds is used.

### 7. Collect carriers

After that the grid is carefully removed with a forceps.

### 8. Immobilization by Negative Staining and ice embedding

Immobilization of the macromolecular complexes, such as the ones shown in Figure 4, on the electron microscopic carrier is carried out by two different techniques - negative staining and cryo-fixation [Harris, J. R. et al. (1991); Dubochet, J. et al. (1982)]. For conventional negative staining after the adsorption of the macromolecular complexes a washing step is performed with two drops of deionized water and subsequently contrast enhancement with two drops of heavy metal. By applying a second preparation technique - cryo fixation - the sample is rapidly frozen. After removing excess fluid by blotting, a thin layer of solution on the electron microscopic grid is obtained. The grid is then plunged into liquid ethane. At a freezing rate of more than 10⁵ K s⁻¹ the aqueous suspension converts to a vitreous phase directly, bypassing the crystalline ice. Thus, the frozen grid will have a thin aqueous layer with macromolecules embedded in an almost natural frozen hydrated environment. After freezing, grids can be kept for a long time at liquid-nitrogen temperatures. These structurally preserved frozen-hydrated samples are then imaged in an electron microscope at liquid-nitrogen or liquid-helium temperatures.

### Example 2

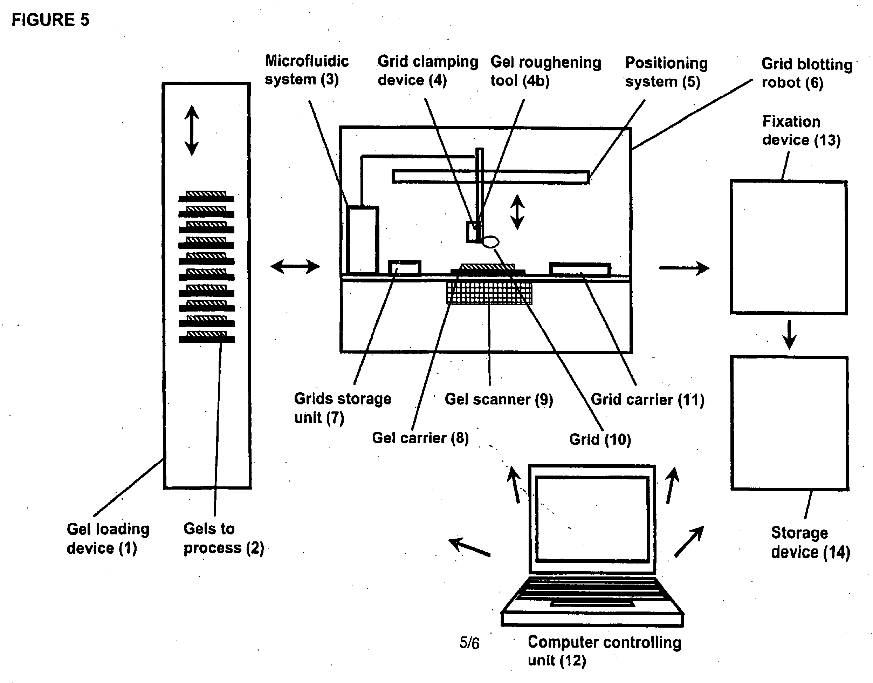

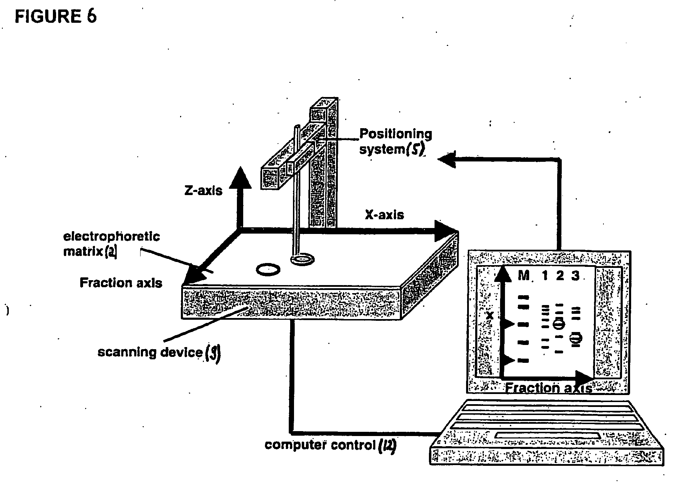

Example 2 in connection with Figures 5 and 6 illustrates one possibility how the method of the present invention can be utilized in an automated process which is contolled by a robot.

### Procedure Robot

The method of the invention can be used for an apparatus performing in an automated way. Referring to figure 5 a gel loading device (1) is connected to a computer controlling unit (12) and transports gels (2) located at gel carriers (8) serially to the grid blotting robot (6). A single, actually processed gel is placed on a gel scanner (9) inside the grid blotting robot (6). After moving the gel to its location the gel is scanned by a gel scanner (9) automatically driven by the computer controlling unit (12). This procedure is shown in detail in figure 6 where the scanning device (2) is attached to the computer control (4) and delivers an image of the electrophoretic matrix (1). The bands of interest can be identified and located by transferring the coordinates of the image of the gel to the coordinates of the positioning system (3), respectively the positioning system (5) shown in figure 5. The positioning system (5) is used to move the gel roughening tool (4b) which is formed like a forceps over the band position investigated by the method. The gel is roughened by the gel roughening tool (4b) in a way that deeper layers of the gel are exposed. After retraction of the gel roughening tool (4b) the positioning system (5) moves the grid clamping device (4) over the grid storage unit (7) and picks one grid. Afterwards the grid is moved by the positioning system (5) in a computer controlled way to the selected position on the gel, respectively the band of interest and is gently placed parallel to the surface of the gel and as described in the method of the present invention. In a next step, optionally, a drop of running buffer solution is added to the grid located on the gel using a microfluidic device (3) directed by the positioning system (5) which is controlled by the computer controlling unit (12). After an incubation time of 60 seconds the grid is picked by the grid clamping device, moved by the positioning system (5) and transferred to a grid carrier (11). From there the grid, respectively multiple processed grids are transferred to a fixation device (13) where the grid is further processed by negative staining or cryo-fixation of the sample. In a next step the fixated grid is moved to a storage device (14) for short or long-term storage of the sample.

### References:

### Blotting methods:

Southern:
   Southern, E. (1975): Detection of specific sequences among DNA fragments separated by gel electrophoresis, J.Mol.Biol. 89, 503
Nitrocellulose:
   Towbin, H. et al. (1976): Electrophoretic transfer of proteins form polyacrylamide gels to nitrocellulose sheets: procedure and some applications. Proc. Natl. Acad. Sci. USA 76, 4350-4354
Nylon:
   Gershoni, J. & Palade, G. (1982): Electrophorecic transfer of proteins from sodium dodecylsulfate-polyacrylamide gels to a positively charged membrane filter. Anal. Biochem. 124, 396-405
PVDF(PolyVinyliDendiFluorid):
   Gültekin, H. & Heermann (1988): The use of polyvinylidendifluorid membranes as a general blotting matrix. Anal. Biochem. 172, 320-329
      Glass-fiber/polybrene
      Vandekerckhove, J. et al. (1985): Proteinblotting on polybrene-coated glass-fiber sheets. Eur. J. Biochem. 152, 9-19
      Eckerskorn, C. et al. (1993): Structural characterization of blotting membranes and the influence of membrane parameters for electroblotting and subsequent aminoacid-sequence analysis of proteins, Electrophoresis 14, 831-838.

### Gel Staining:

Silver staining and coomassie staining:
   Wray, W. et al. (1981): Silver staining of proteins in polyacrylamide gels, Analytic Biochemistry 118, 197-203.
      Merril, C. et al. (1981): Ultrasensitive stain for proteins in polyacrylamide gels shows regional variation in cerebrospinal fluid proteins. Science 211, 1437-1438.
      Williams, L. R. (2001): Staining nucleic acids and proteins in electrophoresis gels, Biotech. Histochem. 76 (3), 127-132.

### Electroelution:

Jeno, P. & Horst, M. (1996): Electroelution of proteins from polyacrylamide gels, 207-214, The protein protocols handbook, Humana Press, Totowa, USA

### Vacuum-dialysis:

Pohl, T. (1990): Concentrations of proteins and removal of solutes. Methods Enzymol. 182, 68-83

### Gel electrophoresis:

Laemmli, U. K. (1970): Cleavage of structural proteins during the assembly of the head of bacteriophage T4. Nature 227, 680-685.
Maurer, H. (1971): Disc electrophoresis and related techniques of polyacrylamide gel electrophoresis. Walter de Gruyter, Berlin/New York
Rickwood, D., Hames, B.D. (1990): Gel electrophoresis of nucleic acids 2nd Edition. IRL Press, Oxford.
Hames, B.D. (1998): Gel electrophoresis of proteins 3rd Edition. Oxford University Press, Oxford.

### Precipitation:

Englard, S., Steifter, S. (1990): Precipitation techniques, Methods Enzymol. 182, 285-300.

### Chromatography methods:

Hagel, L. (1989): Gel filtration, in: Protein purification - principles, high-resolution methods, and applications (Janson, J.-C., Rydén, L., Eds.).VCH, Weinheim. Stellwagen, E. (1990): Gel filtrations, Methods Enzymol. 182, 317-328.
Cutler, P. (1996): Size-exclusion chromatography, Methods Mol. Biol. 59, 269-275.

### Immobilization:

Harris, J. R. & Horne, R. W. (1991): Electron Microscopy in Biology, edited by J. R. Harris, 203-228, IRL Press, Oxford , UK.
Dubochet, J., Lepault, J., Freeman, R., Berriman, J. A. & Homo, J. C. (1982): Electron microscopy of frozen water and aqueous solution, J. Microsc. 128, 219-237.

## Claims

1. A method for the detection and/or analysis of the structure of macromolecular complexes and/or macromolecules comprising the steps of
(a) purifying or separating said macromolecular complexes and/or macromolecules from a sample containing said macromolecular complexes and/or macromolecules by applying in a porous matrix a separation force in a first dimension (X-axis);
(b) transferring in a second dimension (Z-axis) by adsorption forces the macromolecular complexes and/or macromolecules purified or separated in step (a) from the porous matrix onto a carrier wherein said carrier is an electron microscopy grid and contacts the surface of the porous matrix and is positioned parallel to the surface of said matrix and parallel to the direction of the separation force applied in step (a);
(c) immobilizing the macromolecular complexes and/or macromolecules on said carrier after transfer of step (b); and
(d) assessing the structure of the macromolecular complexes and/or macromolecules on said carrier after immobilization of step (c).

2. A method for analyzing whether a subject is afflicted by a disease or prone to developing a disease, wherein said disease is correlated with the presence or absence of protein complexes, and/or virus complexes, and/or bacterial complexes and/or fungal complexes, and/or prion-related complexes comprising the steps of
(a) assessing for the presence and optionally the structure of said protein complexes, and/or virus complexes and/or bacterial complexes and/or fungal complexes and/or prion-related complexes by
(aa) purifying or separating said protein complexes, and/or virus complexes, and/or bacterial complexes, and/or fungal complexes, and/or prion-related complexes from a sample putatively containing said protein complexes, and/or virus complexes and/or bacterial complexes and/or fungal complexes and/or prion-related complexes by applying in a porous matrix a separation force in a first dimension (X-axis);
(ab) transferring in a second dimension (Z-axis) by adsorption forces the protein complexes, and/or virus complexes, and/or bacterial complexes, and/or fungal complexes, and/or prion-related complexes purified or separated in step (aa) from the porous matrix onto a carrier, wherein said carrier is an electron microscopy grid and contacts the surface of the porous matrix and is positioned parallel to the surface of said matrix and parallel to the direction of the separation force applied in step (aa);
(ac) immobilizing the protein complexes, and/or virus complexes, and/or bacterial complexes, and/or fungal complexes, and/or prion-related complexes on said carrier after transfer of step 2(ab); and
(b) correlating the presence or absence or the structure of the protein complexes, and/or virus complexes, and/or bacterial complexes, and/or fungal complexes, and/or prion-related complexes on said carrier with the affliction of said subject by a disease or proneness to developing a disease.

3. The method of claim 2, wherein said subject is a human.

4. The method of any one of claims 1 to 3, further comprising after step 1(a) or step 2(aa) and before step 1(b) or step 2(ab) step (a'), wherein a second separation force is applied in a third dimension (Y-axis) to the macromolecular complexes and/or macromolecules purified or separated in step 1(a) or the protein complexes, and/or virus complexes, and/or bacterial complexes, and/or fungal complexes, and/or prion-related complexes purified or separated in step 2(aa).

5. The method of claim 4, wherein the vector of the second separation force describes an angle in the third dimension to the vector of the separation force of step 1(a) or step 2(aa) in the range between 1 degree and 180 degree.

6. The method of any one of claims 1 to 5, wherein said separation force is generated by an electric field or by gravity.

7. The method according to any one of claims 1 to 6, wherein after step 1(a) or step 2(aa) and before step 1(b) or step 2(ab) the porous matrix is stained and/or scanned.

8. The method of any one of claims 1 to 7, wherein said surface of the porous matrix is roughened.

9. The method of any one of claims 1 to 8, wherein said carrier which contacts the surface of the porous matrix is surrounded by a buffer medium.

10. The method of any one of claims 1 to 9, wherein the transfer in step 1(b) or step 2(ab) is accelerated and the yield of transferred sample is increased by additionally applying an electric field wherein the vector of said electric field is oriented in the second dimension (Z-axis).

11. The method of any one of claims 1 to 10, wherein said porous matrix is a gel or a nanocomposite.

12. The method of claim 11, wherein said gel is a polyacrylmide gel or an agarose gel.

13. The method of claim 11 or 12, wherein said gel is a gradient gel.

14. The method of claim 13, wherein said gradient is in the range of 0,01 %(w/v) to 50%(w/v), preferably in the range of 0,5%(w/v) to 40%(w/v), and more preferably in the range of 1 %(w/v) to 8%(w/v).

15. The method of any one of claims 1 to 14, wherein said electron micoscopy grid is covered by a electron transmitting layer or a holey foil.

16. The method of any one of claims 1 and 4 to 15, wherein said macromolecular complexes are selected from the group consisting of protein complexes, virus particles, eubacteriae, archaebacteriae, organic and anorganic chemical compounds.

17. The method of any one of claims 1 to 16, wherein said protein complexes are native protein complexes.

18. The method of any one of claims 1 to 17, wherein said immobilization in step 1(c) or step 2(ac) is cryo-fixation.

19. The method of any one of claims 1 to 17, wherein said immobilization in step 1(c) or step 2 (ac) is negative staining with heavy atom salts.

20. The method of any one of claims 1 to 19, wherein said assessment of the structure of the macromolecular complexes and/or macromolecules in step 1(d) or said assessment for the presence and optionally the structure of said protein complexes, and/or virus complexes and/or bacterial complexes and/or fungal complexes and/or prion-related complexes in step 2(a) is effected by electron microscopy.

21. The method according to any one of claims 1 to 20, wherein said method is a high throughput method.

22. A device or robot for the detection and/or analysis of the structure of macromolecular complexes and/or macromolecules as defined in any one of claims 1 and 4 to 21 or for the analysis whether a subject is afflicted by a disease or prone to developing a disease as defined in any one of claims 2 to 15 and 17 to 21, wherein said device comprises
(a) a gel loading device (1)
(b) a gel transporter
(c) a grid blotting robot (6) with a grid clamping device (4), a positioning system (5), a gel scanner (9) and optionally a gel roughening tool (4b)
(d) a fixation device (13)
(e) a storage device (14); and
(f) a computer controlling unit (12).

## Patentansprüche

1. Verfahren zum Nachweis und/oder zur Analyse der Struktur von makromolekularen Komplexen und/oder Makromolekülen, umfassend die Schritte
(a) Aufreinigung oder Trennung der makromolekularen Komplexe und/oder Makromoleküle aus einer Probe, welche die makromolekularen Komplexe und/oder Makromoleküle enthält, durch die Anwendung einer Trennkraft in einer porösen Matrix in eine erste Dimension (X-Achse);
(b) Transfer der makromolekularen Komplexe und/oder Makromoleküle, die in Schritt (a) aufgereinigt oder getrennt wurden, durch Adsorptionskräfte von der porösen Matrix auf einen Träger in eine zweite Dimension (Z-Achse), wobei der Träger ein Elektronenmikroskopgitter ist und die Oberfläche der Matrix berührt und parallel zu der Oberfläche der Matrix und parallel zu der Richtung der in Schritt (a) angewandten Trennungskräfte ausgerichtet ist;
(c) Immobilisierung der makromolekularen Komplexe und/oder Makromoleküle auf dem Träger nach dem Transfer von Schritt (b); und
(d) Feststellung der Struktur der makromolekularen Komplexe und/oder Makromoleküle auf dem Träger nach der Immobilisierung von Schritt (c).

2. Verfahren zur Analyse, ob eine Testperson an einer Krankheit leidet oder anfällig ist, eine Krankheit zu entwickeln, wobei die Krankheit korreliert mit dem Auftreten oder Fehlen von Proteinkomplexen und/oder Viruskomplexen und/oder bakteriellen Komplexen und/oder Pilz-Komplexen und/oder Prionen-ähnlichen Komplexen, umfassend die Schritte
(a) Feststellung des Auftretens und gegebenenfalls der Struktur der Proteinkomplexe und/oder Viruskomplexe und/oder bakteriellen Komplexe und/oder Pilz-Komplexe und/oder Prionen-ähnlichen Komplexe durch
(aa) Aufreinigung oder Trennung der Proteinkomplexe und/oder Viruskomplexe und/oder bakteriellen Komplexe und/oder Pilz-Komplexe und/oder Prionen-ähnlichen Komplexe aus einer Probe, die mutmaßlich die Proteinkomplexe und/oder Viruskomplexe und/oder bakteriellen Komplexe und/oder Pilz-Komplexe und/oder Prionen-ähnlichen Komplexe enthält, durch die Anwendung einer Trennkraft in einer porösen Matrix in eine erste Dimension (X-Achse);
(ab) Transfer der Proteinkomplexe und/oder Viruskomplexe und/oder bakteriellen Komplexe und/oder Pilz-Komplexe und/oder Prionen-ähnlichen Komplexe, die in Schritt (aa) aufgereinigt und/oder getrennt wurden, durch Adsorptionskräfte von der porösen Matrix auf einen Träger in eine zweite Dimension (Z-Achse), wobei der Träger ein Elektronenmikroskopgitter ist und die Oberfläche der Matrix berührt und parallel zu der Oberfläche der Matrix und parallel zu der Richtung der in Schritt (aa) angewandten Trennungskräfte ausgerichtet ist;
(ac) Immobilisierung der Proteinkomplexe und/oder Viruskomplexe und/oder bakteriellen Komplexe und/oder Pilz-Komplexe und/oder Prionen-ähnlichen Komplexe auf dem Träger nach dem Transfer in Schritt 2(ab); und
(b) Korrelierung des Auftretens oder Fehlens oder der Struktur der Proteinkomplexe und/oder Viruskomplexe und/oder bakteriellen Komplexe und/oder Pilz-Komplexe und/oder Prionen-ähnlichen Komplexe auf dem Träger mit dem Leiden der Testperson an einer Krankheit oder der Anfälligkeit, eine Krankheit zu entwickeln.

3. Verfahren nach Anspruch 2, wobei die Testperson ein Mensch ist.

4. Verfahren nach einem der Ansprüche 1 bis 3, weiterhin umfassend nach Schritt 1(a) oder Schritt 2(aa) und vor Schritt 1(b) oder Schritt 2(ab) einen Schritt (a'), wobei eine zweite Trennkraft in einer dritten Dimension (Y-Achse) auf die makromolekularen Komplexe und/oder Makromoleküle, die in Schritt 1(a) gereinigt oder abgetrennt wurden, oder die Proteinkomplexe und/oder Viruskomplexe und/oder bakteriellen Komplexe und/oder Pilz-Komplexe und/oder Prionen-ähnlichen Komplexe in Schritt 2(aa) angewandt wird.

5. Verfahren nach Anspruch 4, wobei der Vektor der zweiten Trennkraft in der dritten Dimension einen Winkel zu dem Vektor der Trennkraft von Schritt 1(a) oder Schritt 2(aa) im Bereich von 1 bis 180 Grad aufweist.

6. Verfahren nach einem der Ansprüche 1 bis 5, wobei die Trennkraft durch ein elektrisches Feld oder Schwerkraft erzeugt wird.

7. Verfahren nach einem der Ansprüche 1 bis 6, wobei nach Schritt 1(a) oder Schritt 2(aa) und vor Schritt 1 (b) oder Schritt 2(ab) die poröse Matrix gefärbt und/oder gescannt wird.

8. Verfahren nach einem der Ansprüche 1 bis 7, wobei die Oberfläche der porösen Matrix aufgeraut wird.

9. Verfahren nach einem der Ansprüche 1 bis 8, wobei der Träger, der die Oberfläche der porösen Matrix berührt, von einem Puffermedium umgeben ist.

10. Verfahren nach einem der Ansprüche 1 bis 9, wobei der Transfer in Schritt 1 (b) oder Schritt 2(ab) beschleunigt und der Ertrag der transferierten Probe erhöht wird durch die zusätzliche Anwendung eines elektrischen Feldes, wobei der Vektor des elektrischen Feldes in die zweite Dimension (Z-Achse) ausgerichtet ist.

11. Verfahren nach einem der Ansprüche 1 bis 10, wobei die poröse Matrix ein Gel oder eine Nanoverbundstruktur ist.

12. Verfahren nach Anspruch 11, wobei das Gel ein Polyacrylamidgel oder ein Agarosegel ist.

13. Verfahren nach Anspruch 11 oder 12, wobei das Gel ein Gradientengel ist.

14. Verfahren nach Anspruch 13, wobei der Gradient im Bereich von 0,01%(Gew./Vol.) bis 50%(Gew./Vol.), vorzugsweise im Bereich von 0,5%(Gew./Vol.) bis 40%(Gew./Vol.), und stärker bevorzugt in Bereich von 1 %(Gew./Vol.) bis 8%(Gew./Vol.) liegt.

15. Verfahren nach einem der Ansprüche 1 bis 14, wobei das Elektronenmikroskopgitter bedeckt ist mit einer elektronendurchlässigen Schicht oder einer Folie mit Löchern.

16. Verfahren nach einem der Ansprüche 1 und 4 bis 15, wobei die makromolekularen Komplexe ausgewählt sind aus der Gruppe bestehend aus Proteinkomplexen, Viruspartikeln, Eubakterien, Archaebakterien, organischen und anorganischen chemischen Verbindungen.

17. Verfahren nach einem der Ansprüche 1 bis 16, wobei die Proteinkomplexe natürliche Proteinkomplexe sind.

18. Verfahren nach einem der Ansprüche 1 bis 17, wobei die Immobilisierung in Schritt 1(c) oder Schritt 2(ac) eine Kryofixierung ist.

19. Verfahren nach einem der Ansprüche 1 bis 17, wobei die Immobilisierung in Schritt 1 (c) oder Schritt 2(ac) eine negative Färbung mit Salzen schwerer Atome ist.

20. Verfahren nach einem der Ansprüche 1 bis 19, wobei .die Feststellung der Struktur der makromolekularen Komplexe und/oder der Makromoleküle in Schritt 1 (d) oder die Feststellung des Auftretens und gegebenenfalls der Struktur der Proteinkomplexe und/oder Viruskomplexe und/oder bakteriellen Komplexe und/oder Pilz-Komplexe und/oder Prionen-ähnlichen Komplexe in Schritt 2(a) durch Elektronenmikroskopie erfolgt.

21. Verfahren nach einem der Ansprüche 1 bis 20, wobei das Verfahren ein Verfahren mit hohem Durchsatz ist.

22. Vorrichtung oder Roboter zum Nachweis und/oder zur Analyse der Struktur von makromolekularen Komplexen und/oder Makromolekülen gemäß der Definition in einem der Ansprüche 1 und 4 bis 21 oder für die Analyse, ob eine Testperson an einer Krankheit leidet oder anfällig ist, eine Krankheit zu entwickeln gemäß der Definition in einem der Ansprüche 2 bis 15 und 17 bis 21, wobei die Vorrichtung umfasst:
(a) eine Gelladevorrichtung (1)
(b) einen Geltransporter
(c) einen Netzabsetzroboter (6) mit einem Netzgreifgerät (4), ein Positionierungssystem (5), einen Gelscanner (9) und gegebenenfalls ein Instrument, um Gel aufzurauen (4b)
(d) eine Befestigungsvorrichtung (13)
(e) eine Speichervorrichtung (14); und
(f) eine Computersteuereinheit (12).

## Revendications

1. Procédé de détection et/ou d'analyse de la structure de complexes macromoléculaires et/ou de macromolécules comprenant les étapes consistant à :
(a) purifier ou séparer lesdits complexes macromoléculaires et/ou lesdites macromolécules à partir d'un échantillon contenant lesdits complexes macromoléculaires et/ou lesdites macromolécules en appliquant dans une matrice poreuse une force de séparation dans une première dimension (axe X) ;
(b) transférer dans une deuxième dimension (axe Z) par des forces d'adsorption les complexes macromoléculaires et/ou les macromolécules purifié(e)s ou séparé(e)s dans l'étape (a) depuis la matrice poreuse vers un support, où ledit support est une grille de microscope électronique et est en contact avec la surface de la matrice poreuse et est positionné parallèlement à la surface de ladite matrice et parallèlement à la direction de la force de séparation appliquée dans l'étape (a) ;
(c) immobiliser les complexes macromoléculaires et/ou les macromolécules sur ledit support après le transfert de l'étape (b) ; et
(d) évaluer la structure des complexes macromoléculaires et/ou des macromolécules sur ledit support après l'immobilisation de l'étape (c).

2. Procédé permettant d'analyser si un sujet est affecté par une maladie ou prédisposé à développer une maladie, dans lequel ladite maladie est en corrélation avec la présence ou l'absence de complexes protéiques, et/ou de complexes viraux, et/ou de complexes bactériens et/ou de complexes fongiques, et/ou de complexes à prion comprenant les étapes consistant à
(a) évaluer la présence et facultativement la structure desdits complexes protéiques, et/ou complexes viraux et/ou complexes bactériens et/ou complexes fongiques et/ou complexes à prion
(aa) en purifiant ou en séparant lesdits complexes protéiques, et/ou complexes viraux, et/ou complexes bactériens, et/ou complexes fongiques, et/ou complexes à prion à partir d'un échantillon contenant d'une façon putative lesdits complexes protéiques, et/ou complexes viraux, et/ou complexes bactériens, et/ou complexes fongiques, et/ou complexes à prion en appliquant dans une matrice poreuse une force de séparation dans une première dimension (axe X) ;
(ab) en transférant dans une deuxième dimension (axe Z) par des forces d'adsorption les complexes protéiques, et/ou complexes viraux, et/ou complexes bactériens, et/ou complexes fongiques, et/ou complexes à prion purifiés ou séparés dans l'étape (aa) depuis la matrice poreuse vers un support, où ledit support est une grille de microscope électronique et est en contact avec la surface de la matrice poreuse et est positionné parallèlement à la surface de ladite matrice et parallèlement à la direction de la force de séparation appliquée dans l'étape (aa) ;
(ac) en immobilisant les complexes protéiques, et/ou complexes viraux, et/ou complexes bactériens, et/ou complexes fongiques, et/ou complexes à prion sur ledit support après l'étape de transfert de l'étape 2(ab) ; et
(b) en mettant en corrélation la présence ou l'absence ou la structure des complexes protéiques, et/ou complexes viraux, et/ou complexes bactériens, et/ou complexes fongiques, et/ou complexes à prion sur ledit support avec l'affection dudit sujet par une maladie ou sa prédisposition à développer une maladie.

3. Procédé selon la revendication 2, dans lequel ledit sujet est un humain.

4. Procédé selon l'une quelconque des revendications 1 à 3, comprenant en outre après l'étape 1(a) ou l'étape 2(aa) et avant l'étape 1(b) ou l'étape (2ab) l'étape (a'), dans laquelle une seconde force de séparation est appliquée dans une troisième dimension (axe Y) sur les complexes macromoléculaires et/ou sur les macromolécules purifié(e)s ou séparé(e)s dans l'étape 1(a) ou sur les complexes protéiques, et/ou complexes viraux, et/ou complexes bactériens, et/ou complexes fongiques, et/ou complexes à prion purifiés ou séparés dans l'étape 2(aa).

5. Procédé selon la revendication 4, dans lequel le vecteur de la seconde force de séparation décrit un angle dans la troisième dimension par rapport au vecteur de la force de séparation de l'étape 1(a) ou de l'étape 2(aa) dans la gamme entre 1 degré et 180 degrés.

6. Procédé selon l'une quelconque des revendications 1 à 5, dans lequel ladite force de séparation est produite par un champ électrique ou par la gravité.

7. Procédé selon l'une quelconque des revendications 1 à 6, dans lequel après l'étape 1(a) ou l'étape 2(aa) et avant l'étape 1(b) ou l'étape 2(ab) la matrice poreuse est colorée et/ou numérisée.

8. Procédé selon l'une quelconque des revendications 1 à 7, dans lequel ladite surface de la matrice est rugueuse.

9. Procédé selon l'une quelconque des revendications 1 à 8, dans lequel ledit support qui est en contact avec la surface de la matrice poreuse est entourée d'un milieu tampon.

10. Procédé selon l'une quelconque des revendications 1 à 9, dans lequel le transfert dans l'étape 1(b) ou l'étape 2(ab) est accéléré et le rendement de l'échantillon transféré est augmenté en appliquant en plus un champ électrique dans lequel le vecteur dudit champ électrique est orienté dans la deuxième dimension (axe Z).

11. Procédé selon l'une quelconque des revendications 1 à 10, dans lequel ladite matrice poreuse est un gel ou un nanocomposite.

12. Procédé selon la revendication 11, dans lequel ledit gel est un gel de polyacrylamide ou un gel d'agarose.

13. Procédé selon la revendication 11 ou 12, dans lequel ledit gel est un gel à gradient.

14. Procédé selon la revendication 13, dans lequel ledit gradient est dans la gamme allant de 0,01 % (p/v) à 50 % (p/v), de préférence dans la gamme allant de 0,5 % (p/v) à 40 % (p/v), et de manière davantage préférée dans la gamme allant de 1 % (p/v) à 8 % (p/v).

15. Procédé selon l'une quelconque des revendications 1 à 14, dans lequel ladite grille de microscope électronique est recouverte d'une couche transmettant des électrons ou d'une feuille perforée.

16. Procédé selon l'une quelconque des revendications 1 et 4 à 15, dans lequel lesdits complexes macromoléculaires sont choisis dans le groupe constitué par des complexes protéiques, des particules virales, des eubactéries, des archéobactéries, des composés chimiques organiques et non organiques.

17. Procédé selon l'une quelconque des revendications 1 à 16, dans lequel lesdits complexes protéiques sont des complexes de protéines natives.

18. Procédé selon l'une quelconque des revendications 1 à 17, dans lequel ladite immobilisation dans l'étape 1(c) ou l'étape 2(ac) est une cryofixation.

19. Procédé selon l'une quelconque des revendications 1 à 17, dans lequel ladite immobilisation dans l'étape 1(c) ou l'étape 2(ac) est une coloration négative avec des sels d'atomes lourds.

20. Procédé selon l'une quelconque des revendications 1 à 19, dans lequel ladite évaluation de la structure des complexes macromoléculaires et/ou des macromolécules dans l'étape 1(d) ou ladite évaluation de la présence et facultativement de la structure desdits complexes protéiques, et/ou complexes viraux et/ou complexes bactériens et/ou complexes fongiques et/ou complexes à prion dans l'étape 2(a) est effectuée par microscopie électronique.

21. Procédé selon l'une quelconque des revendications 1 à 20, dans lequel ledit procédé est un procédé de criblage à haut débit.

22. Dispositif ou robot permettant de détecter et/ou d'analyser la structure de complexes macromoléculaires et/ou de macromolécules comme cela est défini dans l'une quelconque des revendications 1 et 4 à 21 ou permettant d'analyser si un sujet est affecté par une maladie ou prédisposé à développer une maladie comme cela est défini dans l'une quelconque des revendications 2 à 15 et 17 à 21, dans lequel ledit dispositif comprend :
(a) un dispositif chargeur de gel (1)
(b) un transporteur de gel
(c) un robot de transfert sur grille (6) avec un dispositif de serrage de grille (4), un système de positionnement (5), un numériseur de gel (9) et facultativement un outil pour rendre le gel rugueux (4b)
(d) un dispositif de fixation (13)
(e) un dispositif de stockage (14) ; et
(f) une unité de contrôle informatique (12).
